# EUROPEAN PATENT APPLICATION

(11) **EP 1 013 271 A2**
(43) Date of publication of application: **28.06.2000**
(21) Application number: 99500240.9
(22) Date of filing: 14.12.1999
(51) Int. Cl.: A61K 9/16, A61K 9/20, A61K 9/50

(54) **Nimodipine tablets**

(30) Priority: 15.12.1998 AR 9801063
(71) Applicant: Gold, Oscar, 1069 Buenos Aires (AR)
(72) Inventor: Gold, Oscar, 1069 Buenos Aires (AR)
(74) Representative: Urizar Anasagasti, José Antonio

(57) **Abstract**

Preparation of a granulated substance in which the characteristic of solubility of Nimodipine is modified. A solution of the drug is prepared with substances that modify the solubility of said drug, said substances being capable of forming a solid solution with said drug. Said solution is absorbed on disintegrant substances and this humid powder is mixed, granulated through a stainless steel mesh (opening 1,5 to 2 mm), and then dried.
**II ―** The particles of the dried granulated substance are put on a large shallow pan where a coating with polymeric substances in solution is applied by aspersion and then it is dried by blowout of hot air
**III ―** Compression of the coated granules in order to obtain tablets containing 60 and 90 mg of Nimodipine.

## Description

### Background of the invention

The present invention refers to a process for the preparation of compositions of programmed liberation, which allow to maintain the therapeutic action of NIMODIPINE (*), active agent liberated by means of said process, with less oscillations of the plasmatic concentrations than the ones occurring with compositions of immediate liberation.
(*): esther 2-methoxyethyl 1-methyl of 1,4-dihydro-2,6-dimethyl-4-(3-nytrophenyl)-3,5-pyridinedicarboxylic acid

The administration of Nimodipine by mouth requires multiple daily doses in virtue of its short average life (0,9 to 1,5 hours), and presents side effects that can include: hypotension, headache, vertigo, vomiting, rash, perspiration, bradycardia, extrasystole and anxiety; therefore **the purpose** of the present invention is to design a formulation of Programmed Liberation of said active agent, to allow the handling of stable plasmatic concentrations, reduce the number of daily doses and to facilitate the adequate control and fulfillment of the treatment on the part of the patient.

### Summary of the invention

Process for the obtention of preparations of programmed liberation of Nimodipine in tablets, which allow the gradual liberation of said active agent and the maintenance of the therapeutic action of said active agent with less oscillations of the plasmatic concentrations.

This invention of tablets containing Nimodipine includes:
**I-** Preparation of a granulated substance in which the characteristic of solubility of Nimodipine is modified, by means of the mixing of the drug with substances that modify the solubility of said drug and also mixing with disintegrant substances. Granulation of said mix, and then drying of the same.
**II ―** Coating by aspersion of the particles of the granules with solutions of polymeric substances.
**III ―** Drying and compression of the coated granules in order to obtain tablets containing 60 and 90 mg of Nimodipine.

### Brief description of the drawings

The object of the invention has been illustrated for better clarity and understanding of the same as an illustrative, not limiting example:

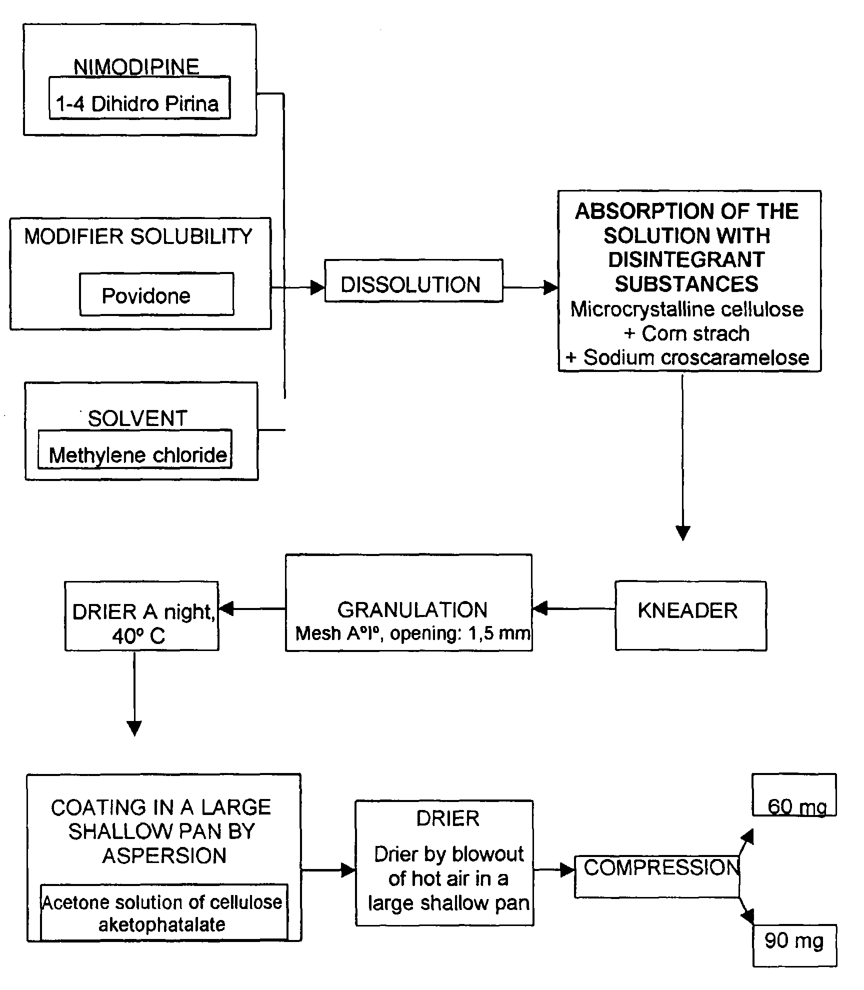

### Detailed description of the preferred embodiments

In the following examples, the embodiment of the present invention is described:

### Example 1: Nimodipine 60 mg

A solution containing 120 g. Nimodipine with 35 g. Povidone was prepared in 200 ml Chloroform. This dissolution was absorbed in 80 g Aerosil 200, and the humid mixture was forced through a mesh of stainless steel with an opening of 2 mm. This granulated substance was dried during one night at 40°C and then the same was compressed in order to obtain tablets of 60 mg. Nimodipine.

### Example 2: Nimodipine 60 mg

100 g of the granulated substance obtained in Example 1, was put on a large shallow pan and 10 ml of an alcoholic solution of Shellac was applied by means of aspersion. This coated granulated substance was dried during one night at 40°C and then it was compressed in order to obtain tablets of 60 mg. Nimodipine.

### Example 3: Nimodipine 90 mg

One Kg. Nimodipine and 2,5 Kg. Povidone were carefully mixed. This mixture was dissolved in 3,5 Lt of Methylene Chloride. On the other part, 4,75 Microcrystalline cellulose, 250 g. Corn starch and 1 Kg. Sodium Croscaramelose were mixed. This mixture of powders was used to absorb the solution of the active principle and Povidone. The moistened powder was granulated through a mesh of stainless steel of 1,5 mm opening, and then it was dried during one night at 40°C. The dry granulated substance was compressed in order to obtain tablets of 90 mg: Nimodipine.

### Example 4: Nimodipine 90 mg

One Kg. of the dried granulated substance obtained in Example 3 was put in a large shallow pan, and 500 ml. of an aketinic solution of Cellulose Aketophtalate was applied by means of aspersion. The granulated substance was coated by hot air blowout in said large shallow pan, then the compression was carried out in order to obtain tablets of 90 mg. Nimodipine.

### Example 5: Nimodipine 60 mg

The coated granulated substance obtained in example 4 was compressed in order to obtain tablets of 60 mg. Nimodipine.
- The tablets thus obtained, verify a ratio in weight of the modifiers of solubility with respect to Nimodipine of 5 to 1 respectively.

### Analysis of the obtained products

The tests of dissolution of the tablets were carried out using the equipment for dissolution of the modified type USP 23, in which sequences of change of the acidity of the medium (pH) in function of time (t) are made, from pH=1 to t= 0 hours, up to pH= 7,2 to t= 8 hours, imitating the physiological sequence:
- 0 to 1 hours:: [C1H] = 0,1 N
- 1 to 2 hours:: pH = 4,5
- 2 to 6 hours:: pH = 6,9
- 6 to 8 hours:: pH = 7,2

The percentages of liberation of the active principle were the following:

| Hours | Example 1 | Example 2 | Example 3 | Example 4 | Example 5 |
|---|---|---|---|---|---|
| 1st. | 12 % | 1% | 91% | 35 % | 37 % |
| 3rd. | 14 % | 3 % | - | 72 % | 74 % |
| 8th. | 35 % | 8 % | - | 82% | 88 % |

Bearing in mind the results in the Examples 4 and 5, tablets of programmed liberation containing 60 mg and 90 mg of Nimodipine were produced, the esther 2-methoxyethyl of 1,4-dihydro-2,6-dimethyl-4-(3-nytrophenyl) -3,5-pyridinedicarboxylic acid, for each tablet. Said preparations were designated as follows:
**SLP® 1:** NIMODIPINE 60 DIFUCAP-EURAND SLP® programmed liberation, 60 mg of NIMODIPINE
**SLP® 2**: NIMODIPINE 90 DIFUCAP-EURAND SLP® programmed liberation, 90 mg of NIMODIPINE

Studies of comparative bioavailability were carried out with respect to a product of conventional or fast liberation present in the local market, employing it as a reference:
**REFERENCE:** NIMOTOP® from BAYER Immediate liberation, 30 mg NIMODIPINE

The studies of bioavailability were carried out in virtue of the collaboration of six healthy male volunteers, and in accordance with the rules of the Declaration of Helsinki. The formulations were administered orally according to the following diagram:

### Dose of 60 mg:

SLP® 1 : A tablet of 60 mg. supplied at 0 hours
REFERENCE: Two compounds of 30 mg. taken once, at 0 hours.

### Dose of 90 mg:

SLP® 2 : A tablet of 90 mg. supplied at 0 hours
REFERENCE : Three compounds of 30 mg. taken once, at 0 hours, 4 hours, 8 hours.

Blood samples were taken from the volunteers at different hours, and the plasmatic concentrations of Nimodipine were analyzed. The statistic meaning of the results was evaluated by ANOVA. The following was carried out with the obtained data:

### Graphs of plasmatic concentration of Nimodipine [N] (ng/ml) vs. time (t) (hours)

### Dose of 60 mg

| Formulation | Max. Plasmatic concentration observed | Time to achieve the maximum concentration |
|---|---|---|
| SLP® 1 | 32,2 ± 3,6 ng/ml | 3,8 ± 0,83 |
| Reference | 50,5 ± 7,3 ng/ml | 1,2 ± 0,17 |

The maximum concentration of SLP® 1 (60 mg) was significantly less than that of the Reference, the maximum time was greater, whilst the Areas under the curves were not statistically different.

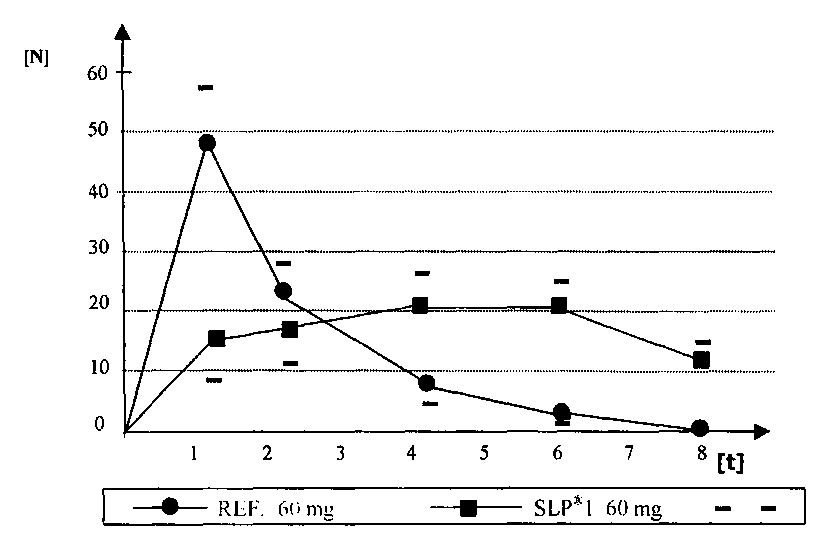

### Dose of 90 mg

| Formulation | Max. Plasmatic concentration observed | Time to achieve the maximum concentration |
|---|---|---|
| SLP® 1 | 30,7 ± 4,7 ng/ml | 4,3 ± 0,3 |
| Reference | 40,0 ± 3,4 ng/ml | 1,0 ± 0,0 |

The maximum concentration of SLP® 2 (90 mg) was not statistically different than that of the 1st. dose (30 mg) of the Reference, the maximum time was significantly greater, and the Areas under the curves were not statistically different.

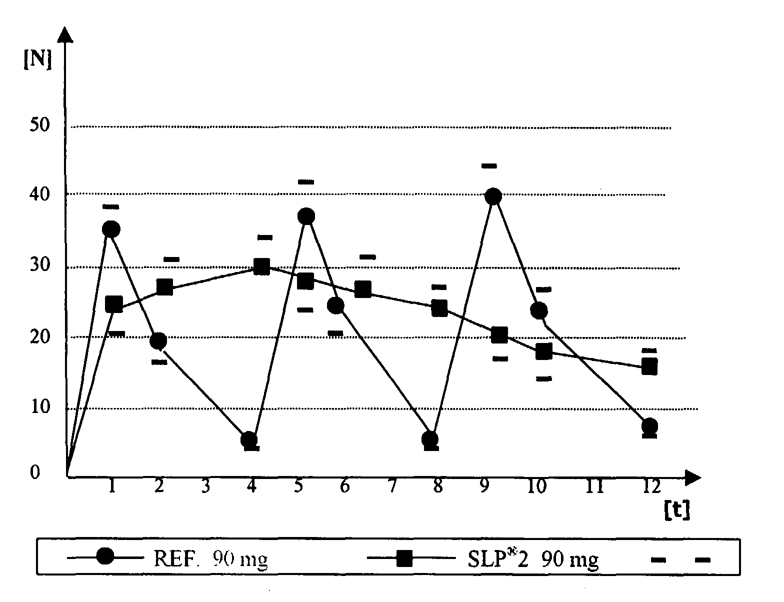

According to the obtained results, the formulation **SLP® 2** (containing 90 mg) is defined as a product of programmed liberation, given that it achieves a maximum plasmatic concentration similar to the Reference (30 mg) and prolongs it as a plateau throughout time.

Having thus described and determined the nature of the present invention, and how it can be carried out, the same is declared to claim as an exclusive right and property:

## Claims

1. A new formulation of tablets of programmed liberation, elaborated with the active principle Nimodipine [esther 2-methoxyethyl 1-methylethyl of 1,4-dihydro-2,6-dimethyl-4-(3-nytrophenyl)-3,5-pyridynedicarboxylic acid], **characterized in that** it achieves the gradual liberation of Nimodipine allowing the maintenance of the therapeutic action of said active principle with less oscillations of the plasmatic concentrations than the ones occurring with formulations of immediate liberation, reducing the number of doses or daily doses and facilitating the control and compliance of the treatment on the part of the patient.
**I-** Preparation of a granulated substance in which the characteristic of solubility of Nimodipine is modified. A solution of the drug is prepared with substances that modify the solubility of said drug, said substances being capable of forming a solid solution with said drug. Said solution is absorbed on disintegrant substances and this humid powder is mixed, granulated through a stainless steel mesh (opening 1,5 to 2 mm), and then dried.
**II ―** The particles of the dried granulated substance are put on a large shallow pan where a coating with polymeric substances in solution is applied by aspersion and then it is dried by blowout of hot air.
**III ―** Compression of the coated granules in order to obtain tablets containing 60 and 90 mg of Nimodipine.

2. Process in accordance with claim 1 wherein the solvents for the dissolution of the drug with the substances modifying the solubility are of the type of Methylene chloride, chloroform and other chlorated derivatives.

3. Process in accordance with claim 1 wherein the substance modifying the solubility is Povidone (PVP).

4. Process in accordance with claim 1 wherein the disintegrant substances are microcrystalline cellulose, Corn starch, sodium croscaramelose or sodium glycolate starch.

5. Process in accordance with claim 1 wherein the polymeric substances for the coating are Cellulose aketophtalate, hydroxypropylmethylcellulose phtalate, derivatives of acrylic acid as Eudragit and Shellac dissolved in alcohol, ketones of chlorated solvents.
